# EUROPEAN PATENT APPLICATION

(11) **EP 4 748 488 A1**
(43) Date of publication of application: **27.05.2026**
(21) Application number: 25305718.6
(22) Date of filing: 20.05.2025
(51) Int. Cl.: B01J 8/06, C07C 9/04

(54) **CATALYTIC METHANATION REACTOR WITH UNIFORM GAS FLOW DISTRIBUTION**

(30) Priority: 20.11.2024 EP 24306937
(71) Applicant: Technip Energies France, 92741 Nanterre Cedex (FR)
(72) Inventor: RONGIER, Clement, 92741 Nanterre Cedex (FR); BARRON, Estefany, 92741 Nanterre Cedex (FR); SIPPEL, Julien, 92741 Nanterre Cedex (FR)
(74) Representative: Edson, Russell Gregory

(57) **Abstract**

Disclosed is a catalytic methanation tubular reactor including a distribution member and one or more associated flow guides, which are collectively usable to produce a uniform feed gas flow distribution at inlet ends of an array of flow channels that at least partially extend through a shell of the catalytic methanation tubular reactor. The distribution member and the one or more flow guides can be positioned in a header volume between a header inlet and the inlet ends of the flow channels, with the one or more flow guides subjacent to the distribution member. A plurality of distribution openings may extend through the distribution member. The one or more flow guides may bound the distribution openings. By controlling the size and distribution of the distribution openings and using the flow guides, a feed gas flow can be uniformly distributed across the inlet end array of the catalytic methanation tubular reactor.

## Description

### Technical Field

The present disclosure relates generally to gas processing vessels, and more particularly although not necessarily exclusively, to a catalytic methanation reactor and to optimizing a gas flow therein.

### Background

Fluid processing vessels, such as heat exchangers and gas reactors, may utilize a configuration in which multiple fluid pathways extend within or through a shell-side fluid flow path of a vessel shell. For example, in a shell and tube heat exchanger, a plurality of tubes may extend within a shell, a first fluid can travel through the tubes, and a second fluid can travel through the shell and over the tubes, whereby heat can be transferred from the first fluid to the second fluid, or vice versa. Certain fluid processing vessels, such as tubular gas reactors (e.g., catalytic methanation tubular reactors), may also utilize a shell and tube configuration where a plurality of tubes extend through a shell. In such a tubular gas reactor, a catalyst may be located in the tubes, and a fluid in the form of a reactant gas mixture (e.g., carbon monoxide/dioxide mixed with hydrogen) can undergo a catalyst facilitated reaction while traveling through the tubes to generate a desired end product (e.g., methane). Heat generated by the reaction may be transferred through the tube walls to a cooling fluid flowing through the shell, which can help control the temperature of the reaction and avoid overheating the tubes and the catalyst. The efficiency of this heat transfer can be increased by improving the uniformity of the fluid flow rate distribution through the tubes.

### Summary

As used below, any reference to a series of examples is to be understood as a reference to each of those examples disjunctively (e.g., "Examples 1-4" or "Example No. 1 to Example No. 4" is to be understood as "Examples 1, 2, 3, or 4").

Example No. 1 is a catalytic methanation reactor including a header having a header inlet and a header wall that defines a header volume in fluid communication with the header inlet. A plurality of fluid flow channels extend downstream from a first support member residing in the header volume. The fluid flow channels are in fluid communication with the header volume at inlet openings of the fluid flow channels, which are arranged in an inlet opening array. A distribution member is located in the header volume between and spaced apart from both the header inlet and the inlet opening array and includes a plurality of distribution openings. A flow guide extends between the inlet opening array and the distribution member, and narrows a portion of the header volume between the distribution member and the inlet opening array. A catalyst is located in at least some of the plurality of fluid flow channels to facilitate a reaction of a feed gas that is flowable through the at least some of the plurality of fluid flow channels.

Example No. 2 is Example No. 1, wherein the catalytic methanation reactor is intended to be orientated to be substantially vertical with the distribution member above the first support member, and wherein fluid is intended to flow downwards through the distribution member and into the inlet openings of the fluid flow channels so as to flow down along a longitudinal axis of the fluid flow channels.

Example No. 3 is Example No. 1, wherein the flow guide is axially parallel with a longitudinal axis of the fluid flow channels and extends between the distribution member and the first support member.

Example No. 4 is Example No. 1, wherein the flow guide has opposing longitudinal ends, wherein each longitudinal end is in contact with an internal wall of the header.

Example No. 5 is Example No. 1, wherein the fluid flow channels are tubes and the reactor has a no-tube-in-window configuration where the inlet opening array resides in a tube region of the first support member that is aligned with the header inlet and is bounded on two opposed sides by window regions having no tubes.

Example No. 6 is Example No. 1, wherein a trough is defined in the header volume between the flow guide, first support member and the header wall.

Example No. 7 is Example No. 5, wherein the trough is in axial alignment with a no-tube-in-window region of the first support member.

Example No. 8 is Example No. 1, wherein the inlet opening array comprises a first planar array of openings, wherein the distribution openings are arranged in a second planar array of openings, and wherein the flow guide comprises at least one wall extending between the first and second planar arrays.

Example No. 9 is Example No. 8, wherein the support member is a planar member, the first planar array of openings is located in the support member, and the distribution member is a plate or a planar sheet.

Example No. 10 is Example No. 9, wherein the support member comprises a first central region including the first planar array of openings and at least one first peripheral region in which no inlet openings are located, and the at least one wall extends from a boundary between the first central region and the first peripheral region. Also, the distribution member comprises a second central region including the distribution openings and at least one second peripheral region in which no distribution openings are located, wherein the at least one wall extends from a boundary between the second central region and the second peripheral region.

Example No. 11 is Example No. 10, wherein the plurality of distribution openings in the distribution member are all uniform in size and are distributed uniformly across the second central region of the distribution member.

Example No. 12 is Example No. 10, wherein at least some of the plurality of distribution openings in the distribution member are non-uniform in size, distribution openings of a smaller size are concentrated near a central area of the second central region of the distribution member, and distribution openings of a larger size are concentrated near a periphery of the second central region of the distribution member.

Example No. 13 is Example No. 9, wherein the fluid flow channels are tubes and the support member is a tube sheet.

Example No. 14 is Example No. 1, wherein the header is an elliptical header.

Example No. 15 is Example No. 1, wherein the feed gas is a mixture comprising at least carbon monoxide and hydrogen, carbon dioxide and hydrogen, or carbon monoxide, carbon dioxide, and hydrogen, and the catalyst is a metal selected from the group consisting of nickel, ruthenium, rhodium, and palladium.

Example No. 16 is a catalytic methanation tubular reactor including a shell defining a shell-side fluid flow path, and a plurality of tubes that extend at least partially through the shell-side fluid flow path, where each tube includes an inlet end and the inlet ends are arranged in an inlet end array located in a tube support member. The catalytic methanation tubular reactor also includes an elliptical header having a header inlet, and a header wall that at least partially defines a header volume, which is in fluid communication with the inlet end array. The catalytic methanation tubular reactor additionally includes a distribution member having a plurality of distribution openings. The distribution member is positioned in the header volume between the header inlet and the tube support member so as to be spaced apart from the inlet end array. One or more flow guides extend between the distribution member and the tube support member and at least partially bound the distribution openings in the distribution member and the inlet ends of the plurality of tubes. A catalyst is located in at least some of the plurality of tubes to facilitate a reaction of a feed gas that is flowable through the at least some of the plurality of tubes.

Example No. 17 is Example No. 16, wherein the tube support member is a tube sheet having a no-tube-in-window configuration where the inlet end array resides in a tube region of the tube sheet that is aligned with the header inlet and is bounded on two opposed sides by window regions having no tubes.

Example No. 18 is Example No. 16, wherein the one or more flow guides extends between the distribution member and the tube support member.

Example No. 19 is Example No. 16, wherein the one or more flow guides are planar.

Example No. 20 is Example No. 16, wherein the one or more flow guides are axially parallel with a longitudinal axis of the tubes.

Example No. 21 is Example No. 16, wherein the one or more flow guides have opposing longitudinal ends, wherein each longitudinal end is in contact with the header wall.

Example No. 22 is Example No. 16, wherein a trough is defined in the header volume between a respective one of said one or more flow guides, the tube support member and an internal wall of the header.

Example No. 23 is Example No. 17, wherein the plurality of distribution openings in the distribution member are all uniform in size and are distributed uniformly across a distribution region of the distribution member that is aligned with the header inlet and the tube region.

Example No. 24 is Example No. 17, wherein at least some of the plurality of distribution openings in the distribution member are non-uniform in size, distribution openings of a smaller size are concentrated near a central area of a distribution region of the distribution member that is aligned with the header inlet and the tube region, and distribution openings of a larger size are concentrated near a periphery of the distribution region of the distribution member.

Example No. 25 is Example No. 24, wherein the one or more flow guides comprise a pair of spaced apart flow guides, and each flow guide of the pair of spaced apart flow guides extends along the distribution member such that opposite ends of each flow guide are in abutting contact with an inner wall of the header.

Example No. 26 is Example No. 25, wherein the feed gas is a mixture comprising at least carbon monoxide and hydrogen, carbon dioxide and hydrogen, or carbon monoxide, carbon dioxide, and hydrogen, and the catalyst is a metal selected from the group consisting of nickel, ruthenium, rhodium, and palladium.

Example No. 27 is a method that includes introducing a feed gas flow into a header volume of a header of a catalytic methanation reactor. The method also includes passing the feed gas flow through a plurality of distribution openings of a distribution member positioned within the header volume and located between and spaced apart from both a header inlet and an inlet end array comprising inlet ends of a plurality of flow channels that extend through a shell-side fluid flow path within a shell of the catalytic methanation reactor. The method additionally includes directing the feed gas flow from the distribution member to the inlet end array through a narrowed portion of the header volume defined at least in part by one or more flow guides that extend between the distribution member and the inlet end array and at least partially bound the distribution openings in the distribution member and the inlet ends of the plurality of flow channels. The method further includes generating methane by contacting the feed gas flow with a catalyst located in at least some of the plurality of flow channels.

Example No. 28 is Example No. 27, wherein the catalytic methanation reactor is orientated to be substantially vertical in use, e.g. with the header inlet uppermost.

Example No. 29 is Example No. 27, wherein the feed gas flows downwards from the header inlet and down along the longitudinal axis of the flow channels.

Example No. 30 is Example No. 27, wherein the plurality of distribution openings are all uniform in size and are uniformly distributed across a distribution region of the distribution member, or at least some of the plurality of distribution openings are nonuniform in size and are nonuniformly distributed across the distribution region of the distribution member.

Example No. 31 is any one of Example No. 27 to Example No. 30, wherein the one or more flow guides include a pair of spaced apart flow guides, and each flow guide of the pair of spaced apart flow guides extends along the distribution member such that opposite ends of each flow guide are in abutting contact with an inner wall of the header.

Example No. 32 is any one of Example No. 27 to Example No. 31, wherein the feed gas is a mixture comprising at least carbon monoxide and hydrogen, carbon dioxide and hydrogen, or carbon monoxide, carbon dioxide, and hydrogen, and the catalyst is a metal selected from the group consisting of nickel, ruthenium, rhodium, and palladium.

Example No. 33 is any one of Example No. 27 to Example No. 32, wherein a flow rate of the feed gas flow at the inlet ends of the flow channels is no more than 110% and no less than 90% of a target nominal feed gas flow rate.

### Brief Description of the Drawings

FIG. 1 is a cross-sectional view of a hemispherical header portion of a vessel having a shell and tube configuration, according to one example.
FIG. 2 is an isometric section view of the header portion shown in FIG. 1.
FIG. 3A is an isometric view of a distribution member shown to be installed in the hemispherical header portion in FIGS. 1-2, according to one example.
FIG. 3B is a right-side elevation view of the distribution member shown in FIG. 3A.
FIG. 4A is a flow simulation illustrating a fluid flow within a volume of the hemispherical header portion of FIG. 1 when no distribution member is present, according to one example.
FIG. 4B is a flow simulation illustrating a normalized fluid flow velocity distribution across an array of the tubes of the vessel of FIGS. 1-2 that corresponds to the header volume fluid flow shown in FIG. 4A.
FIG. 5A is a flow simulation illustrating a fluid flow within a volume of the hemispherical header portion of FIG. 1 when a distribution member without flow guides is present, according to one example.
FIG. 5B is a flow simulation illustrating a normalized fluid flow velocity distribution across the array of the tubes of the vessel of FIGS. 1-2 that corresponds to the header volume fluid flow shown in FIG. 5A.
FIG. 6A is a flow simulation illustrating a fluid flow within a volume of the hemispherical header portion of FIG. 1 when a distribution member with flow guides is present, according to one example.
FIG. 6B is a flow simulation illustrating a normalized fluid flow velocity distribution across the array of the tubes of the vessel of FIGS. 1-2 that corresponds to the header volume fluid flow shown in FIG. 6A.
FIG. 7 is a cross-sectional view of an elliptical header portion of another vessel having a shell and tube configuration, according to one example.
FIG. 8 is an isometric section view of the header portion shown in FIG. 7.
FIG. 9A is a flow simulation illustrating a fluid flow within a volume of the elliptical header portion of FIGS. 7-8 when no distribution member is present, according to one example.
FIG. 9B is a flow simulation illustrating a normalized fluid flow velocity distribution across an array of the tubes of the vessel of FIGS. 7-8 that corresponds to the header volume fluid flow shown in FIG. 9A.
FIG. 10A is a flow simulation illustrating a fluid flow within the volume of the elliptical header portion of FIGS. 7-8 when a distribution member with flow guides is present, according to one example.
FIG. 10B is a flow simulation illustrating a normalized fluid flow velocity distribution across the array of the tubes of the vessel of FIGS. 7-8 that corresponds to the header volume fluid flow shown in FIG. 10A.
FIG. 11 is an isometric view of a distribution member arranged on an underlying tube support member, according to one example.
FIG. 12 is a transparent top view of the distribution member and underlying tube sheet arrangement shown in FIG. 11.
FIG. 13 is an isometric section view of an elliptical header portion of another vessel having a shell and tube configuration, according to one example.
FIG. 14 is an isometric view of a distribution member shown to be installed in the elliptical header portion in FIG. 13, according to one example.
FIG. 15A is a flow simulation illustrating a fluid flow within the volume of the elliptical header portion of FIG. 13 when the distribution member of FIG. 14 is present.
FIG. 15B is a flow simulation illustrating a normalized fluid flow velocity distribution across the array of the tubes of the vessel of FIG. 13 that corresponds to the header volume fluid flow shown in FIG. 15A.

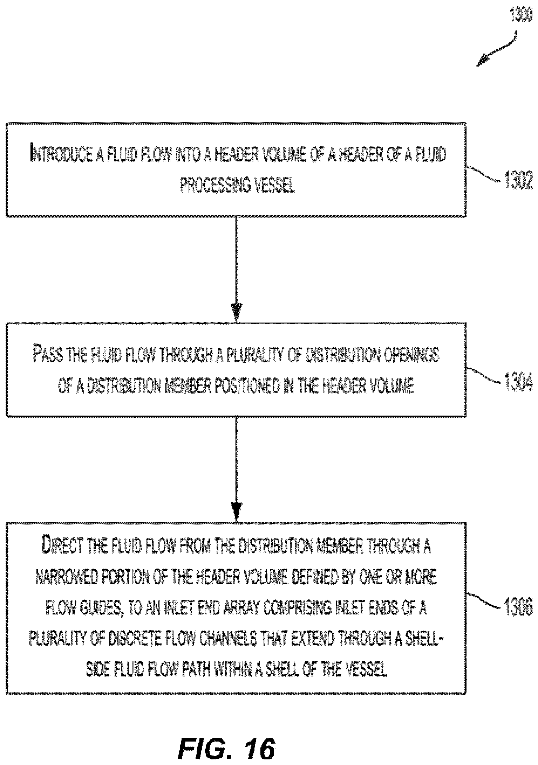

FIG. 16 is a flowchart illustrating a method of uniformly distributing a fluid flow across the inlets to the tubes of a vessel having a shell and tube configuration, according to one example.

### Detailed Description

Certain aspects and examples of the present disclosure relate to fluid processing vessels such as heat exchangers or gas reactors (e.g., tubular gas reactors) equipped with a distribution member and an associated one or more flow guides that are collectively usable to produce a uniform or substantially uniform fluid flow distribution at an inlet end of an array of flow channels that at least partially extend through a shell of the vessel. Substantially uniform fluid flow distribution may include, for instance, a fluid flow distribution in which a fluid flow rate at each of the inlet ends is no more than 110% and no less than 90% of a nominal fluid flow rate. In the absence of such a distribution member, it has been discovered that the fluid flow rate into the flow channels of such a vessel that are directly under or near a header inlet may be substantially greater than the fluid flow rate into the flow channels that are situated farther away from the header inlet.

In some examples, improving the uniformity of the fluid flow rate distribution may create a more balanced and less turbulent circulation of the fluid within a header volume that is defined by a header wall and a flow channel support member in which inlet ends of the flow channels (e.g., tubes) are arrayed. In some examples, the vessel may be a shell and tube heat exchanger or a tubular gas reactor where a first fluid flows through the tubes and a second fluid flows through the shell and over the tubes. **In** the case of a shell and tube heat exchanger, a more uniform distribution of the fluid flow rate through the tubes can increase the efficiency of the heat exchange process. **In** the case of a tubular gas reactor (e.g., a catalytic methanation tubular reactor), a more uniform distribution of the fluid flow through the tubes can improve the efficiency of a reaction or other process that is meant to occur within the tubes, and may avoid degradation of a catalyst located in the tubes, tube damage that might otherwise result from overheating (hotspots) at the catalyst locations, and overheating of the catalyst.

**In** examples where the vessel is a shell and tube heat exchanger or tubular gas reactor, the tubes may be arranged in what is commonly referred to as a no-tube-in-window configuration. In a no-tube-in-window configuration, the plurality of tubes that extend into or through the shell are arranged such that no tubes are present near an inner wall of the shell in at least some areas. The areas within the shell that are devoid of tubes may be referred to as window regions, as may, for example, corresponding areas of components such as a tube support member that are installed to the vessel.

According to one example shell and tube vessel configuration, a distribution member can be positioned in the header volume between the header inlet and a tube support member in which inlet ends of the tubes are retained in an inlet end array. In some examples, the distribution member can be substantially flat and can have a first side and an opposed second side. The distribution member may have a peripheral shape that matches a cross-sectional shape of the header into which the distribution member will be installed. A plurality of distribution openings may extend through the distribution member from the first side to the second side. Each distribution opening may define an opening area, and the totality of the distribution openings can define a total opening area of the distribution member. The opening area of the distribution member can vary along the distribution member to better equalize distribution of a fluid flow therethrough. The distribution openings may be arranged along at least a portion of the distribution member to define a distribution region. For example, when the shell and tube vessel is a shell and tube heat exchanger or a tubular gas reactor having a no-tube-in-window configuration, the distribution region may be restricted to an area of the distribution member that will overlie a tube (e.g., central) region of the tube support member, with no distribution openings present in the areas (i.e., window regions) of the distribution member that will overlie the window regions of the tube support member.

In an example, only part of the peripheral shape of the distribution member matches a cross-sectional shape of the header into which the distribution member will be installed, so that fluid may flow beyond the unmatched part of the peripheral shape (e.g. towards the tube support member) without passing through the distribution openings. For example, where the header has a circular or elliptical cross-section, the peripheral shape of the distribution member may have opposing arcs which match the cross-sectional shape of the header, but wherein parts of the peripheral shape between the arcs are inwardly spaced from the header. Hence, in some examples, one or more portions of the distribution member do not extend all the way to the header wall, so that fluid may flow beyond the part of the peripheral shape which does not extend to the header wall without passing through the distribution openings.

One or more flow guides may also be located in the header volume between the distribution member and the tube support member. The one or more flow guides may bound the distribution openings in the distribution member. The one or more flow guides may extend between the distribution member and the tube support member. The one or more flow guides may be planar. The one or more flow guides may extend axially parallel with a longitudinal axis of the flow channels (e.g. a longitudinal axis of the vessel).

In some examples, the one or more flow guides may comprise a pair of spaced apart flow guides that extend between the distribution member and the tube support member. The one or more flow guides has opposing longitudinal ends, wherein each longitudinal end is in contact with an internal wall of the header. **In** some examples, a trough is defined in the header volume between a respective one of said one or more flow guides, the support member and the header wall (e.g. in the segment defined between a flow guide and the header wall).

In other examples, the one or more flow guides may be a continuous single flow guide that extends between the distribution member and the tube support member and encircles the distribution openings. The single flow guide may define a ring or annulus radially inboard of the header wall and within which the distribution openings are bounded. In some examples, a trough is defined in the header volume between said flow guide, the support member and the header wall (e.g. radially outboard of the flow guide).

In some examples, the distribution member and the one or more flow guides may be separate components. In other examples, the one or more flow guides may be affixed to or may otherwise be an integral part of the distribution member. A height dimension of the one or more flow guides may define a distance of separation of the distribution member from the tube support member and the inlet end array exposed therein. The one or more flow guides may also extend along the second side of the distribution member such that opposite ends of each of the one or more flow guides abut the inner wall of the header. The presence of the one or more flow guides can narrow the portion of the header volume between the distribution member and the tube support member and thereby restrict a path of a fluid that has passed through the distribution openings in the distribution member to the tube region of the tube support member.

Hence, the one or more flow guides may define a narrowed volume of the header volume between the distribution member and the tube support member. In some examples, the whole periphery of the distribution member is in contact with the header wall. In other examples, the distribution member may have a configuration in which not all part of the periphery are in contact with the header wall. Rather, the header volume may define at least one trough below the level of the distribution member, each trough delimited by a respective flow guide, the tube support member, and the header wall.

In some examples, the shell and tube vessel is intended to be orientated to be vertical. That is to say, the longitudinal axis of the flow channels (e.g. the tubes) may be orientated to be vertical. In such an example, the header inlet is above the distribution member and fluid is intended to flow downwards from the header inlet, through the distribution openings, and into the tube openings (so as to flow downwards through the flow channels in their vertical orientation).

In some examples, the vessel may have a hemispherical header. In other examples, the vessel may have an elliptical header (i.e. a header of elliptical cross section). The shell of vessel will typically have a circular cross-sectional shape, but the use of shells having other cross-sectional shapes is possible and the peripheral shape of the distribution member may be correspondingly configured.

As described above, a substantially uniformly distributed fluid flow at the flow channel inlet ends of a heat exchanger or tubular gas reactor can be achieved by installing an example distribution member with one or more flow guides into the fluid flow path within a header portion of the heat exchanger or tubular gas reactor. The distribution member may operate to provide a uniform fluid flow across the inlet ends of the flow channels (e.g., tubes) by, for example, slowing the velocity of the fluid flow through a portion of the distribution member that underlies or is closer to the header inlet. Altering the fluid flow velocity in this manner can facilitate a substantially uniformly distributed fluid flow at the inlet ends of the flow channels of a heat exchanger or tubular gas reactor and, when present, through a catalyst located into the flow channels.

In some examples, the distribution openings in the distribution member may be uniform in size and may be uniformly distributed across the distribution region of the distribution member. In other examples, the distribution openings may be uniform in size but non-uniformly distributed across the distribution region of the distribution member. For example, there may be a lower frequency of occurrence of the distribution openings in an area of the distribution member that underlies or is closer to the header inlet, and a higher frequency of occurrence of the distribution openings in areas of the distribution member that are farther away from the inlet (e.g., the frequency of occurrence of the distribution openings may increase as the distance from the header inlet increases). In still other examples, the distribution openings may be non-uniform in size and the size of the distribution openings may increase as the distance from the header inlet increases. In some examples, the distribution openings may all have the same shape, while in other examples, at least some of the distribution openings may be of dissimilar shape. In some examples, the distribution member may be made of the same material as the shell, the header, and/or the flow channel support member of a fluid processing vessel such as a heat exchanger or tubular gas reactor to which the distribution member will be installed.

Illustrative examples follow and are given to introduce the reader to the general subject matter discussed herein rather than to limit the scope of the disclosed concepts. The following sections describe various additional features and examples with reference to the drawings in which like numerals indicate like elements, and directional descriptions are used to describe the illustrative aspects, but, like the illustrative aspects, should not be used to limit the present disclosure.

Examples of a fluid processing vessel can include an enclosure through which flows a first fluid, and one or more flow channels that extend within the enclosure and through the flow path of the first fluid. A second fluid can flow through the one or more flow channels, such that heat can be transferred from one fluid to another without a comingling of the fluids. In some examples, the fluid processing vessel may employ a cross or counter flow design, where the fluid flowing through the enclosure and the fluid flowing through the flow channels travel in opposite directions.

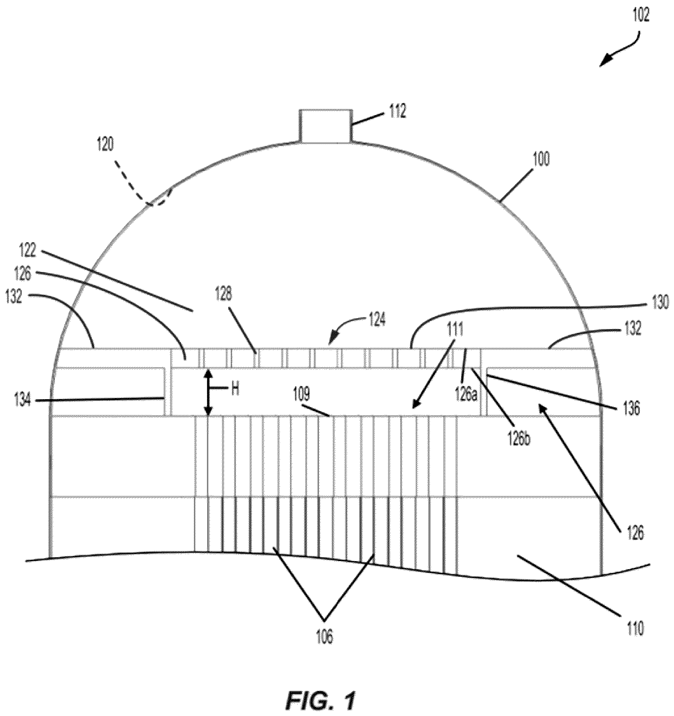

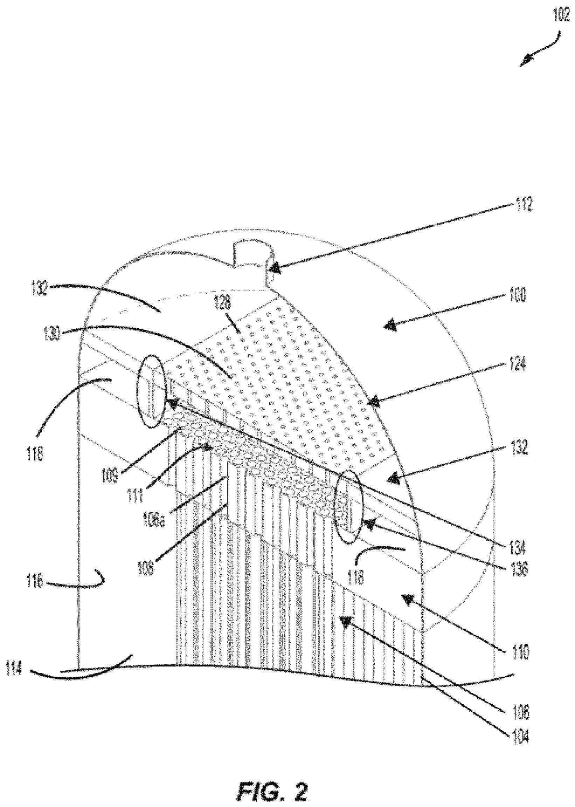

FIG. 1 is a cross-sectional view and FIG. 2 is an isometric section view of one example of a hemispherical header portion 100 of a fluid processing vessel 102 having an enclosure configured as a shell 104 and a number of flow channels configured as a collection of tubes 106. Thus, the fluid processing vessel 102 has a shell and tube configuration and may function as, for example, a heat exchanger or a tubular gas reactor. Openings of the tubes 106 may be arranged as a planar array of openings. More particularly, inlet ends 106a of the tubes 106 may be retained in tube retention holes 108 (see FIG. 2) of a tube (e.g., central) region 109 of a tube support member 110 (e.g., a tube sheet) to form an inlet end array 111. In some examples, the tube support member 110 may be a planar sheet and may be affixed to the shell 104. While not shown in FIGS. 1-2, such a configuration may also include a similar tube support member to retain outlet ends of the tubes 106 near an outlet end of the fluid processing vessel 102. The tube support member 110 can be seen to seal against an inner wall 116 of the shell 104 to prevent the shell-side fluid flow from communicating with the inlet ends 106a of the tubes 106.

A header inlet 112 admits a fluid into the fluid processing vessel 102 to flow through the tubes 106. The fluid may be a gas or a liquid.

In certain examples, the fluid processing vessel 102 is intended to be orientated to be substantially vertical, e.g. with the header inlet 112 uppermost. That is to say, the tubes 106 may be orientated to be perpendicular to the surface on which the fluid processing vessel 102 is located. In such an example, fluid is intended to flow downwards through the distribution member and into the tube openings (so as to flow down along the longitudinal axis of the tubes 106).

When the fluid processing vessel 102 is a heat exchanger, a temperature of the fluid flowing through the tubes 106 may be increased or decreased by a flow of fluid that enters the shell 104 via a separate inlet and travels through the shell 104 (i.e., as a shell-side fluid flow) and over the tubes 106. When the fluid processing vessel 102 is a tubular gas reactor, a catalyst may be located in the tubes and the fluid directed into the header inlet 112 may be a feed gas. The feed gas may undergo a catalyst facilitated reaction while flowing through the tubes 106 to produce a desired end product. A shell-side fluid flow over the tubes 106 may help to control the reaction temperature and may help to cool the product(s) of the reaction prior to discharge thereof from the tubular gas reactor.

As shown in FIGS. 1-2, the fluid processing vessel 102 has a no-tube-in-window configuration in which the tubes 106 are arranged such that at least some areas (window regions) 114 near the inner wall 116 of the shell 104 are devoid of the tubes 106. The tube support member 110 likewise has corresponding solid peripheral (window) regions or areas 118 that are devoid of the tube retention holes 108.

As may be best observed from FIG. 1, the header 100 has an inner header wall 120 that defines a header volume 122. The header volume 122 is in fluid communication with inlet end array 111 of the tubes 106. Thus, a fluid entering the header inlet 112 can flow into the header volume 122 and then into and through the tubes 106.

According to one example configuration, a distribution member 124 can be positioned in the header volume 122 between the header inlet 112 and the inlet end array 111 to improve the distribution of the fluid flow across the inlet ends 106a of the tubes 106. In this example of the fluid processing vessel 102, wherein the inlet end array 111 is arranged in the tube support member 110, the distribution member 124 is positioned between the header inlet 112 and the tube support member 110. In some examples, especially examples where the distribution member 124 is retrofit into an existing vessel header, the distribution member 124 can be fixed in the header volume 122 by fasteners (e.g., threaded fasteners), by welding, etc.

As shown, the distribution member 124 may be substantially flat (e.g., may be a plate or a planar sheet) and may include a first side 124a and an opposed second side 124b. The first side 124a of the distribution member 124 will face the header inlet 112 when the distribution member 124 is located in the header volume 122, while the second side will face the tubes 106. **The** distribution member 124 may have a peripheral shape that matches a cross-sectional shape of the header 100 and may seal against or be sealed to the header wall 120.

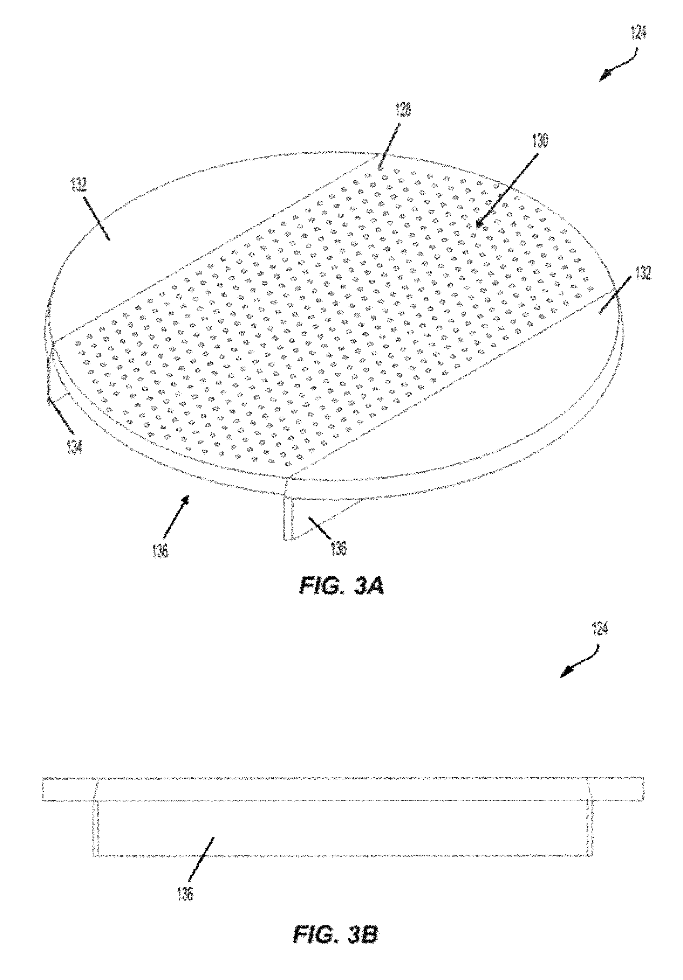

Referring now also to FIGS. 3A-3B, which illustrate an isometric view and a right side elevation view, respectively, of the distribution member 124, it may be observed that a plurality of distribution openings 128 extend through the distribution member 124 from the first side 124a to the second side 124b. Each distribution opening 128 defines an opening area, and the plurality of distribution openings 128 collectively define an overall opening area of the distribution member 124. **The** opening area of the distribution member 124, and the sizes and arrangement of the individual distribution openings 128, determines the fluid flow capacity and flow distribution of the distribution member 124. **In** some examples, the size(s) and arrangement of the individual distribution openings 128 may be a function of the header volume 122 (or another header or vessel dimension), the desired fluid flow velocity, and/or a desired pressure drop through the distribution member 124.

**The** area of the distribution member 124 across which the distribution openings 128 are arranged may be restricted to define a distribution region 130 (e.g., a central region) of the distribution member 124. **In** this example of a shell and tube heat exchanger or tubular gas reactor having a no-tube-in-window configuration, the distribution region 130 may be restricted to an area of the distribution member 124 that will overlie the tube region 109 of the tube support member 110 and the inlet end array 111 of the tubes 106 located therein upon installation of the distribution member 124 in the header volume 122. In such an example, no distribution openings are present in the areas (i.e., window or peripheral regions) 132 of the distribution member 124 that will overlie the window or peripheral regions 118 of the tube support member 110.

One or more flow guides 134 136 may also be located in the header volume 122 extending between the distribution member 124 and the tube support member 110. The flow guides 134, 136 are planar and are arranged to be axially parallel with a longitudinal axis of the tubes 106.

The one or more flow guides 134 136 may bound the distribution openings 128. For example, as shown herein, the one or more flow guides 134 136 may comprise a pair of spaced apart flow guides 134, 136 that extend between the distribution member 124 and the tube support member 110. As can be seen, each flow guide has opposing longitudinal ends, wherein each longitudinal end is in contact with the wall 120 of the header 100. A height H of the flow guides 134, 136 may define a separation distance of the distribution member 124 from the tube support member 110 and the inlet ends 106a of the tubes 106. As may be most clearly observed in FIG. 3B, the flow guides 134, 136 may extend along the second side 124b of the distribution member 124 such that opposite ends of each flow guide 134, 136 will abut the header wall 120 when the distribution member 124 is installed in the header volume 122. The flow guides 134, 136 may be arranged on the distribution member 124 so that, for example, one flow guide 134 extends along a boundary between the distribution region 130 and one of the window regions 132 of the distribution member 124, and the other flow guide 136 extends along a boundary between the distribution region 130 and the other window region 132 of the distribution member 124. The flow guides may thus bound opposite sides of the distribution region 130 of the distribution member 124. Likewise, the flow guides 134, 136 may simultaneously bound opposite sides of the inlet end array 111 in the tube support member 110. For example, one flow guide 134 may extend along a boundary between the tube region 109 and one of the window (peripheral) regions 118 of the tube support member 110, and the other flow guide 136 may extend along a boundary between the tube region 109 and the other window (peripheral) region 118 of the tube support member 110. The flow guides 134, 136 can thus partially define a narrowed flow path for fluid that has passed through the distribution member 124.

In another example (not shown), a single flow guide may extend between the distribution member 124 and the tube support member 110, and a height dimension of the flow guide may define a distance of separation of the distribution member 124 from the tube support member 110 and the inlet end array 111 exposed therein. In such an example, the single flow guide may be arranged on the distribution member 124 so that, for example, the single flow guide surrounds the distribution openings 128 in the distribution region 130 of the distribution member 124. For example, the single flow guide may trace a circular or rectangular path along the distribution member 124 to surround the distribution openings 128 and to partially define a flow path for fluid that has passed through the distribution member 124.

Upon a fluid flow entering the header 100 and passing through the distribution openings 128 in the distribution member 124, the subsequent path of the fluid flow can be restricted by the one or more flow guides 134 136, in cooperation with the header wall 120, to the tube region 109 of the underlying tube support member 110 where the inlet end array 111 is exposed. This can help to more uniformly distribute the fluid flow to the tubes 106 and can impede or prevent a non-beneficial flow of the fluid to the window regions 118 of the tube support member 110.

In some examples, the distribution member 124 and the one or more flow guides 134, 136 may be separate components. In other examples, the one or more flow guides 134 136 may be affixed to or may otherwise be an integral part of the distribution member 124. For example, the one or more flow guides 134 136 may be affixed to the second side 124b of the distribution member 124 or the one or more flow guides 134 136 may be integral portions of the distribution member 124 that project outwardly from the second side 124b thereof toward the tube support member 110.

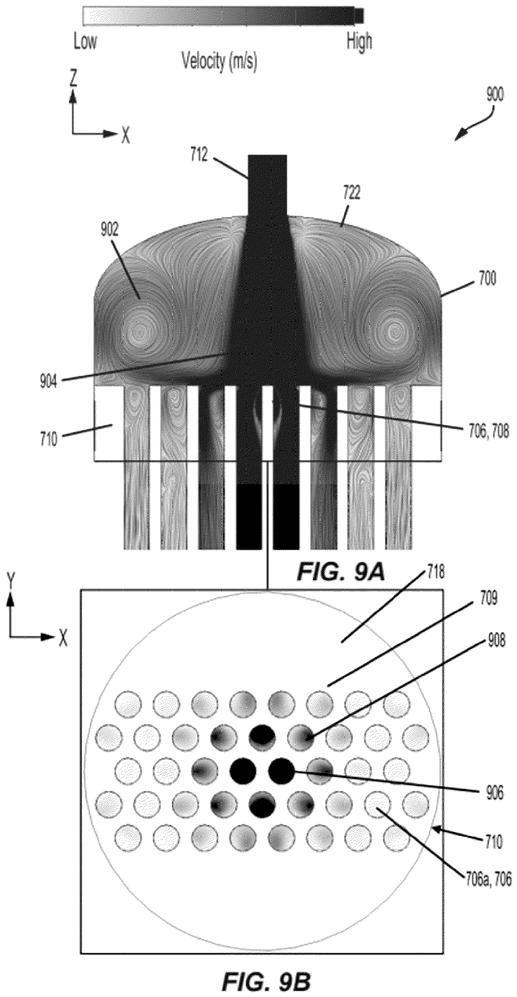

In some examples, and as illustrated FIG. 2 and FIG. 3A, the distribution openings 128 of the distribution member 124 may be uniform in size and may be uniformly arranged (e.g., linearly arrayed) across the distribution region 130 of the distribution member 124. As described below and illustrated in FIGS. 9-11, the distribution openings 128 may be of non-uniform size in other examples.

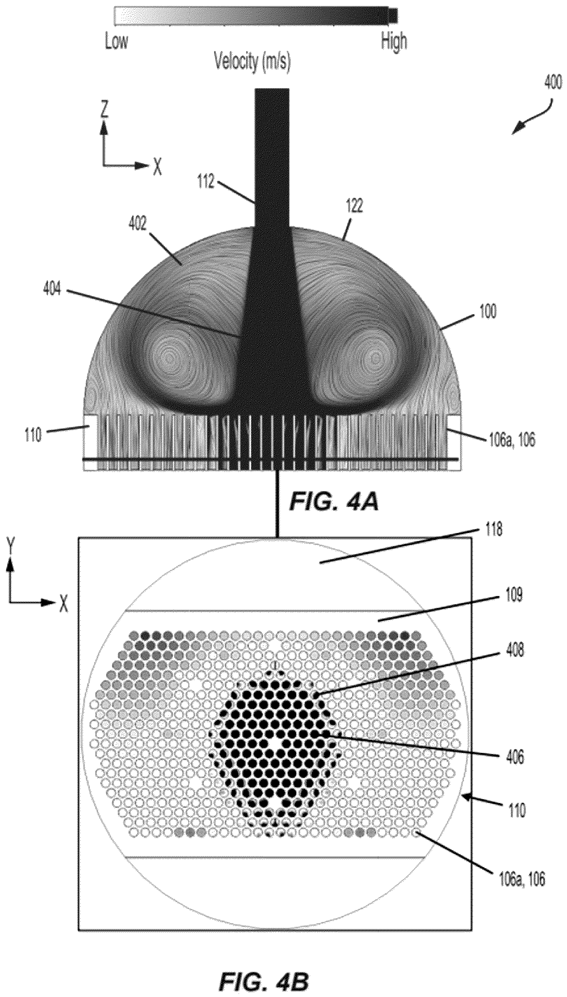

FIG. 4A is a flow simulation 400 illustrating a fluid flow within the header volume 122 of the hemispherical header 100 of FIGS. 1-2 according to an example where neither the distribution member 124 nor the one or more flow guides 134 136 is present. Both a fluid circulation pattern and associated fluid velocities within the header 100 are depicted in FIG. 4A. FIG. 4B is a mapping of the corresponding normalized fluid flow velocity at the inlet end 106a of each tube 106 of the array of tubes 106 supported in the tube region 109 of the tube support member 110.

As can be understood by reference to the legend provided in FIG. 4A, introducing a fluid flow into the header 100 of the fluid processing vessel 102 without the distribution member 124 or the one or more flow guides 134 136 installed therein results in a fluid circulation 402 within the header volume 122 having a high velocity portion 404 that is not well-aligned with the tube region 109 of the tube support member 110. Additionally, FIG. 4B shows that the normalized fluid flow velocity at the inlet ends 106a of the tubes 106 is significantly maldistributed across the tube region 109. For example, there is an area 406 of undesirably high normalized fluid flow velocity near the right-central portion of the tube region 109, which is substantially encircled by a much smaller area 408 of undesirably low normalized fluid flow velocity. The normalized fluid flow velocity distribution across the remaining portions of the tube region 109 is also shown to be non-uniform. As previously explained, this can result in a number of problems, whether the fluid processing vessel 102 is a heat exchanger or a tubular gas reactor.

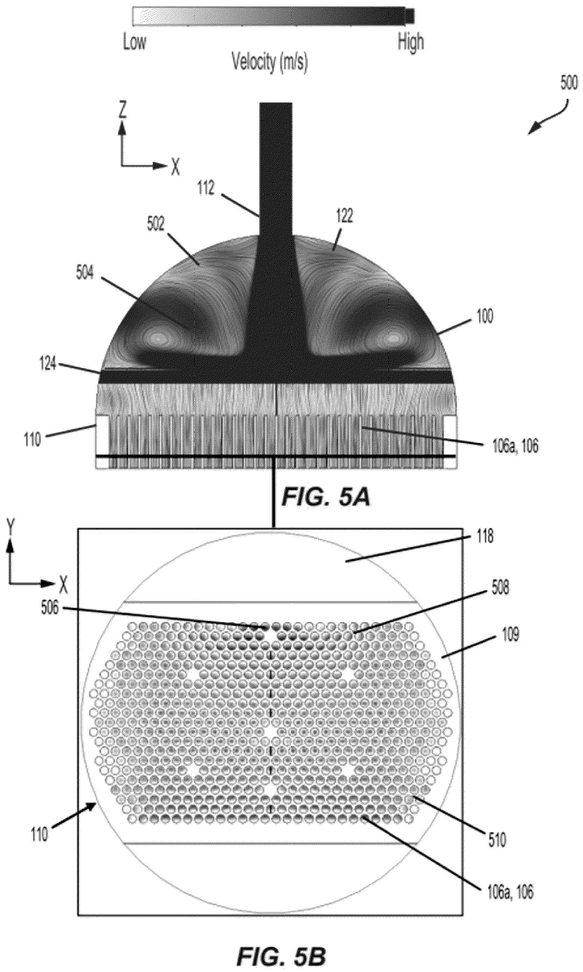

FIG. 5A is a flow simulation 500 illustrating a fluid flow within the header volume 122 of the hemispherical header 100 of FIGS. 1-2 according to an example where the distribution member 124 is installed in the header volume 122 without the one or more flow guides 134, 136. In a like manner to FIG. 4A, both a fluid circulation pattern and associated fluid velocities within the header 100 are depicted in FIG. 5A. FIG. 5B is a mapping of the corresponding normalized fluid flow velocity at the inlet end 106a of each tube 106 of the array of tubes 106 supported in the tube region 109 of the tube support member 110.

As can be understood by reference to the legend provided in FIG. 5A, introducing a fluid flow into the header 100 of the fluid processing vessel 102 when the distribution member 124 is installed therein but the one or more flow guides 134 136 is absent, results in a fluid circulation 502 within the header volume 122 where a high velocity portion 504 is better-aligned with the tube region 109 of the tube support member 110 than in the example vessel configuration associated with the flow simulation of FIG. 4A. However, as depicted in FIG. 5B, the fluid flow velocity at the inlet ends 106a of the tubes 106 still exhibits a degree of maldistribution across the tube region 109. Particularly, there is a limited area 506 of undesirably high normalized fluid flow velocity near the left-central portion of the tube region 109, and other high normalized fluid flow velocity areas 508, 510 near and along an opposite side of the tube region 109.

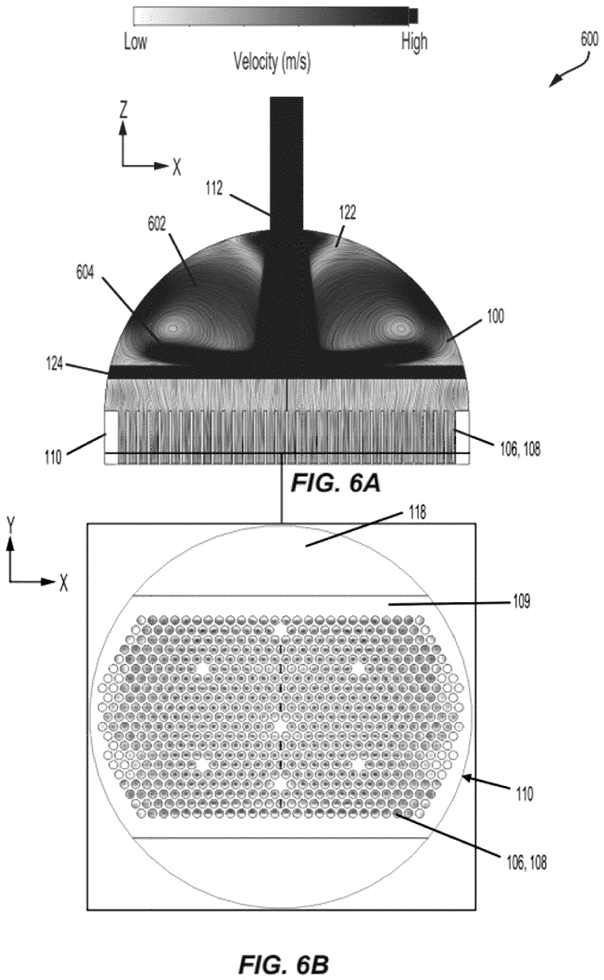

FIG. 6A is a flow simulation 600 illustrating a fluid flow within the header volume 122 of the hemispherical header 100 of FIGS. 1-2 according to an example where the distribution member 124 and the one or more flow guides 134, 136) are both installed in the header volume 122 as depicted in FIGS. 1-2. In a like manner to FIGS. 4A and 5A, both a fluid circulation pattern and associated fluid velocities within the header 100 are depicted in FIG. 6A. FIG. 6B is a mapping of the corresponding normalized fluid flow velocity at the inlet end 106a of each tube 106 of the array of tubes 106 supported in the tube region 109 of the tube support member 110.

As can be understood by reference to the legend provided in FIG. 6A, in comparison to the configuration example of FIG. 5A, introducing a fluid flow into the header 100 of the fluid processing vessel 102 with distribution member 124 and the one or more flow guides 134 136 both installed therein can result in a fluid circulation 602 within the header volume 122 that has a more uniform overall flow velocity, as well as a further improved alignment of a high velocity portion 604 of the fluid flow with the tube region 109 of the tube support member 110. Additionally, FIG. 6B shows that the normalized fluid flow velocity at the inlet ends 106a of the tubes 106 may be uniformly distributed across substantially the entire tube region 109.

The flow simulations of FIGS. 4A-4B, 5A-5B, and 6A-6B clearly demonstrate that fluid flow distribution uniformity is improved as a result of locating the distribution member 124 and the one or more flow guides 134 136 in the header volume 122 as shown and described. For example, with the distribution member 124 and the one or more flow guides 134 136 both installed in the header volume 122, fluid flow modeling has shown that a deviation of the fluid flow rate through the tubes 106 can be limited to no more than 110% and no less than 90% of a target nominal fluid flow rate. In addition to improving heat transfer efficiency, limiting the amount of fluid flow rate deviation through the tubes 106 can minimize resulting changes in the temperature of the fluid flowing through the tubes 106.

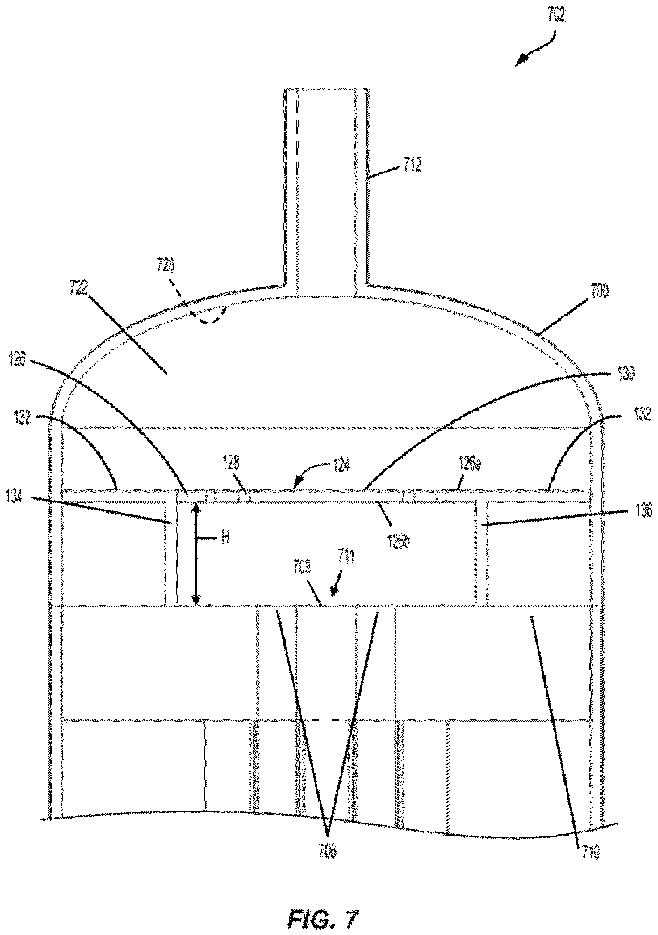

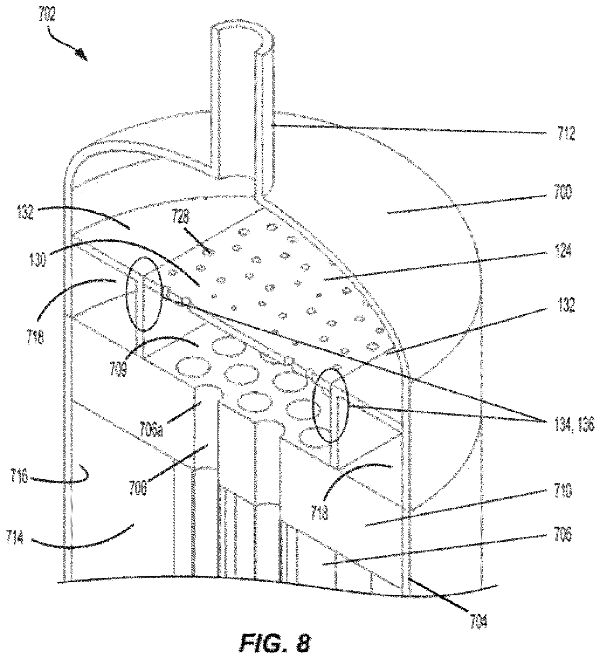

FIG. 7 is a cross-sectional view and FIG. 8 is an isometric section view of an elliptical header portion 700 (i.e. a header portion of elliptical cross section) of another fluid processing vessel 702 having a shell 704 and tube 706 configuration, according to one example. In this particular example, the fluid processing vessel 702 is a tubular gas reactor, and more particularly, a catalytic methanation tubular reactor. The catalytic methanation tubular reactor 702 can be used to produce methane through the catalytic hydrogenation of a reactant gas such as carbon monoxide and/or carbon dioxide. In the catalytic methanation tubular reactor 702, a metal catalyst, such as nickel, ruthenium, rhodium, or palladium can be placed in the tubes 706 at a fixed location. In some examples, the metal catalyst can be configured as a metallic active phase dispersed on a porous or non-porous support. In some examples, the catalyst may be metal particles or pellets that create fixed reactor beds within the tubes 706. A feed gas stream comprising a mixture of carbon monoxide and/or carbon dioxide and hydrogen can be introduced to the reactor and caused to flow through the tubes. The feed gas stream can undergo a catalyst-facilitated reaction while flowing through the tubes, which generates methane and water.

In the catalytic methanation tubular reactor 702, controlling the flow rate of the feed gas stream through each tube 706 is particularly important in order to avoid catalytic (reactor) bed degradation, and to maintain the methanation reaction temperature within limits that will avoid generating hot spots in the tubes 706. The feed gas flow velocity cannot be adjusted once the feed gas enters a tube, thus it is desirable to ensure that a uniform gas flow distribution exists at inlet ends 706a of the tubes 706.

The catalytic methanation tubular reactor 702 may have more tubes 706 than what is shown in FIGS. 7-8. However, a lesser number of tubes 706 is shown in this example of the catalytic methanation tubular reactor 702 to demonstrate that a distribution member and a flow guide(s) as described in the present disclosure can be used to produce a more uniform distribution of the fluid flow into fluid processing vessels of multiple different configurations. In the catalytic methanation tubular reactor 702, inlet ends 706a of the tubes 706 may be retained in tube retention holes 708 (see FIG. 8) of a tube region 709 of a tube support member 710 (e.g., a tube sheet) to form an inlet end array 711. In some examples, the tube support member 710 may be affixed to the shell 704. While not shown in FIGS. 7-8, such a configuration would also include a similar tube support member to retain outlet ends of the tubes 706 near an outlet end of the catalytic methanation tubular reactor 702. The tube support member 710 can be seen to seal against an inner wall 716 of the shell 704 to prevent the shell-side fluid flow from communicating with the inlet ends 706a of the tubes 706.

A header inlet 712 admits fluid (e.g., feed gas) into the catalytic methanation tubular reactor 702 to flow through the tubes 706. The temperature of the reaction can be controlled, at least in part, by a simultaneous flow of fluid that enters the shell 704 via a separate inlet and travels through the shell 704 (i.e., as a shell-side fluid flow) and over the tubes 706.

As shown in FIGS. 7-8, the catalytic methanation tubular reactor 702 has a no-tube-in-window configuration in which the tubes 706 are arranged such that at least some areas (window regions) 714 near the inner wall 716 of the shell 704 are devoid of the tubes 706. The tube support member 710 likewise has corresponding solid areas (window regions) 718 that are devoid of the tube retention holes 708.

As may be best observed from FIG. 7, the header 700 has an inner header wall 720 that defines a header volume 722. The header volume 722 is in fluid communication with inlet end array 711 of the tubes 706. Thus, a fluid entering the header inlet 712 can flow into the header volume 722 and then into and through the tubes 706.

A distribution member can be positioned in the header volume 722 between the header inlet 712 and the inlet ends 706a of the array of tubes 706. The distribution member used in this example of the catalytic methanation tubular reactor 702 is the same distribution member 124 described above. Thus, FIGS. 3A-3B and the associated description provided above may be referenced with respect to understanding the construction and operation of the distribution member 124 relative to the catalytic methanation tubular reactor 702.

In this example of the catalytic methanation tubular reactor 702, wherein the inlet end array is arranged in the tube support member 710, the distribution member 124 is positioned between the header inlet 712 and the tube support member 710. In some examples, especially examples where the distribution member 124 is retrofit into an existing vessel header, the distribution member 124 can be fixed in the header volume 722 by fasteners (e.g., threaded fasteners), by welding, etc.

One or more flow guides can also be located in the header volume 722 between the distribution member 124 and the tube support member 710. The one or more flow guides used in this example of the catalytic methanation tubular reactor 702 is the same as the one or more flow guides 134 136 described above. Thus, FIGS. 1-3B and the associated description provided above may be referenced with respect to understanding the construction and operation of the one or more flow guides 134 136 relative to the catalytic methanation tubular reactor 702.

Upon a fluid flow (e.g., feed gas steam) entering the header 700 and passing through the distribution openings 128 in the distribution member 124, the subsequent path of the fluid flow can be restricted by the one or more flow guides 134 136, in cooperation with the header wall 720, to the tube region 709 of the underlying tube support member 710 where the inlet end array 711 is exposed. This can help to more uniformly distribute the fluid flow to the tubes 706 and can impede or prevent a non-beneficial flow of the fluid to the window regions 718 of the tube support member 710.

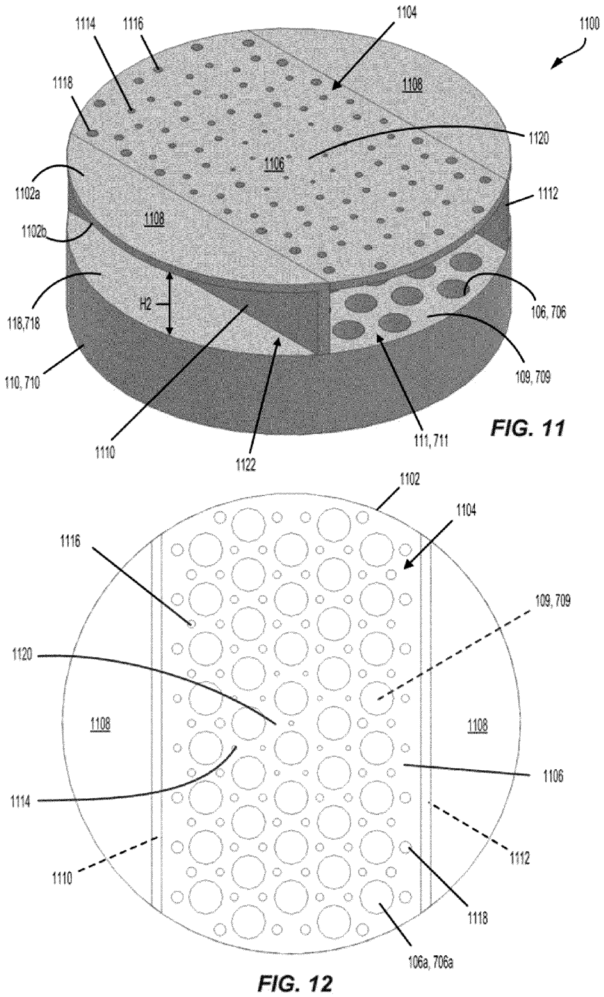

In some examples, and as illustrated FIG. 8 and FIG. 3A, the distribution openings 128 may be uniform in size and may be uniformly arranged (e.g., linearly arrayed) across the distribution region 130 of the distribution member 124. As described below and illustrated in FIGS. 11-12, the distribution openings 128 may be of non-uniform size in other examples.

FIG. 9A is a flow simulation 900 illustrating a fluid flow within the header volume 722 of the elliptical header 700 of FIGS. 7-8 according to an example where neither the distribution member 124 nor the one or more flow guides 134 136 is present. Both a fluid circulation pattern and associated fluid velocities within the header 700 are depicted in FIG. 9A. FIG. 9B is a mapping of the fluid corresponding normalized fluid flow velocity at the inlet end 706a of each tube 706 of the array of tubes 106 supported in the tube region 709 of the tube support member 710.

As can be understood by reference to the legend provided in FIG. 9A, introducing a fluid flow into the header 700 of the catalytic methanation tubular reactor 702 without the distribution member 124 or the one or more flow guides 134 136 installed therein results in a fluid circulation 902 within the header volume 722 having a high velocity portion 904 that is narrowly concentrated on a limited area of the tube region 709 of the tube support member 710. As illustrated by FIG. 9B, the normalized fluid flow velocity at the inlet ends 706a of the tubes 706 is thus significantly maldistributed across the tube region 709. For example, there is an area 906 of undesirably high normalized fluid flow velocity at substantially a central point of the tube region 709, which corresponds to the area of the tube region 709 that underlies and is aligned with the header inlet 712. The area 906 of undesirably high normalized fluid flow velocity is substantially encircled by a smaller area 908 of undesirably low normalized fluid flow velocity. The normalized fluid flow velocity distribution across the remaining portions of the tube region 709 is also shown to be non-uniform. As previously explained, his can result in a number of problems, whether the catalytic methanation tubular reactor 702 is a heat exchanger or a gas processing column (e.g., a catalytic methanation tubular reactor).

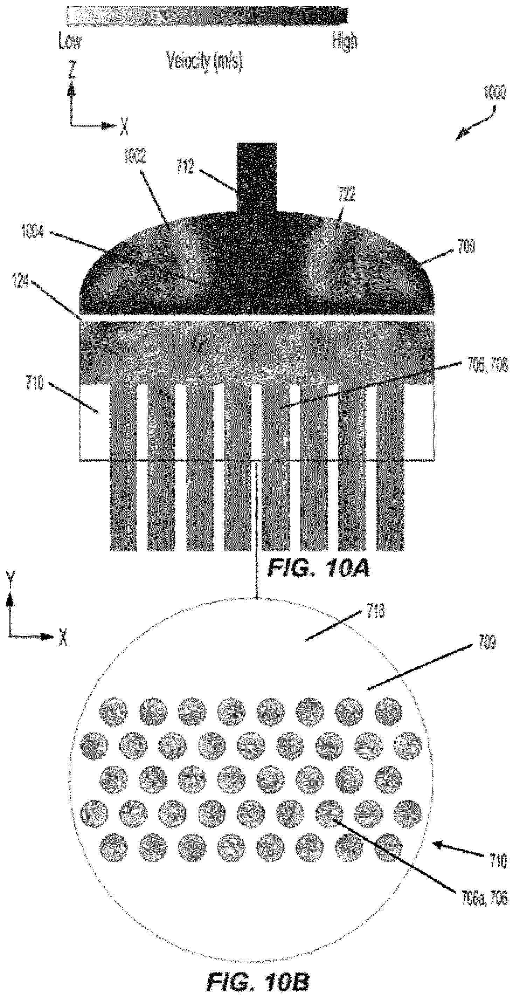

FIG. 10A is a flow simulation 1000 illustrating a fluid flow pattern and fluid flow velocities within the header volume 722 of the elliptical header 700 of FIGS. 7-8 according to an example where the distribution member 124 and the one or more flow guides 134, 136 are both installed in the header volume 722 as depicted in FIGS. 7-8. In a like manner to FIG. 9A, both a fluid circulation pattern and associated fluid velocities within the header 700 are depicted in FIG. 10A. FIG. 10B is a mapping of the corresponding normalized fluid flow velocity at the inlet end 706a of each tube 706 of the array of tubes 706 supported in the tube region 709 of the tube support member 710.

As can be understood by reference to the legend provided in FIG. 10A, in comparison to vessel configuration associated with the flow simulation of FIG. 9A, introducing a fluid flow into the header 700 of the catalytic methanation tubular reactor 702 with the distribution member 124 and the one or more flow guides 134 136 both installed therein can result in a fluid circulation 1002 within the header volume 722 that has a more uniform overall flow velocity. Additionally, the high velocity portion 1004 of the fluid flow may be distributed over a larger area of the tube region 709 of the tube support member 710. As may be observed in FIG. 10B, the normalized fluid flow velocity at the inlet ends 706a of the tubes 706 is uniformly distributed across substantially the entire tube region 709.

The flow simulations of FIGS. 9A-9B and 10A-10B clearly demonstrate that fluid flow distribution uniformity is improved as a result of locating the distribution member 124 and the one or more flow guides 134 136 in the header volume 722 of a tubular gas reactor as shown and described. For example, with the distribution member 124 and the one or more flow guides 134 136 both installed in the header volume 722, fluid flow modeling has shown that a deviation of the fluid flow through the tubes 706 can be limited to no more than 110% and no less than 90% of a target nominal fluid flow rate. In addition to improving heat transfer efficiency, limiting the amount of fluid flow rate deviation through the tubes 706 can minimize resulting changes in the temperature of the fluid flowing through the tubes 706.

FIG. 11 is an isometric view of another example of a distribution member 1100 that may be used in the fluid processing vessel 102 of FIGS. 1-2 or the catalytic methanation tubular reactor 702 of FIGS. 7-8 instead of the distribution member 124. To that end, the distribution member 1100 is shown in FIG. 11 to be arranged relative to the underlying tube support member 110 of the fluid processing vessel 102 of FIGS. 1-2 or the tube support member 710 of the catalytic methanation tubular reactor 702 of FIGS. 7-8.

The distribution member 1100 may be similar in construction to the distribution member 1100 of FIGS. 3A-3B. For example, the distribution member 1100 may have a first side 1102a and an opposed second side 1102b. The first side 1102a of the distribution member 1100 will face the header inlet 112, 712 when the distribution member 1100 is installed in the header volume 122 of the fluid processing vessel 102 of FIGS. 1-2 or the header volume 722 of the catalytic methanation tubular reactor 702 of FIGS. 7-8, while the second side 1102b will face the tubes 106, 706 of the fluid processing vessel 102, 702. The distribution member 1100 may have a peripheral shape that matches a cross-sectional shape of the header 100, 700 to which it is installed and may seal against or be sealed to a header wall 120, 720.

A plurality of distribution openings 1104 may extend through the distribution member 1100 from the first side 1102a to the second side 1102b. Each distribution opening 1104 defines an opening area, and the plurality of distribution openings 1104 collectively define an overall opening area of the distribution member 1100. The opening area of the distribution member 1100, and the sizes and arrangement of the individual distribution openings 1104, determines the fluid flow capacity and flow distribution of the distribution member 1100. In some examples, the size(s) and arrangement of the individual distribution openings 1104 may be a function of the header volume (or another header or vessel dimension), the fluid flow velocity, and/or a pressure drop through the distribution member 1100.

The area of the distribution member 1100 across which the distribution openings 1104 are arranged may be restricted to define a distribution region 1106 of the distribution member 1100. In this example, the distribution region 1106 may be restricted to an area of the distribution member 1100 that will, upon installation of the distribution member 1100, overlie the tube region 109 of the tube support member 110 of FIGS. 1-2 and the inlet end array 111 of the tubes 106 located therein, or the tube region 709 of the tube support member 710 and the inlet end array 711 of the tubes 706 located therein. In such an example, no distribution openings are present in the areas (i.e., window regions) 732 of the distribution member 1100 that will overlie the window regions 718 of the tube support member 710.

The distribution member 1100 may also be associated with a one or more flow guides 1122 that can be located in the header volume 122, 722 between the distribution member 1100 and the tube support member 110, 710. The one or more flow guides 1122 may bound the distribution openings 1104. For example, the one or more flow guides 1122 may comprise a pair of spaced apart flow guides 1110, 1112 that extend between the distribution member 1100 and the tube support member 110, 710. In this example, the flow guides 1110, 1112 are planar and are intended to extend vertically (e.g. parallel with a longitudinal axis of the tubes 106).

A height H2 of the flow guides 1110, 1112 may define a separation distance of the distribution member 1100 from the tube support member 110, 710 and the inlet end arrays 111, 711 exposed therein. The flow guides 11110, 1112 may extend along the second side 1102b of the distribution member 1100 such that opposite ends of each flow guide 1110, 1112 will abut the header wall 120, 720 when the distribution member 1100 is installed in the header volume 122, 722. The flow guides 1110, 1112 may be arranged on the distribution member 1100 so that, for example, one flow guide 1110 resides between the distribution region 1106 and one of the window regions 1108 of the distribution member 1100, and the other flow guide 1112 resides between the distribution region 1106 and the other window region 1108 of the distribution member 1100. The flow guides 1110, 1112 may thus bound opposite sides of the distribution region 1106 and can partially define a flow path for fluid that has passed down through the distribution member 1100 (from the header inlet 112, 712 which is uppermost when the catalytic methanation tubular reactor 102 is in a vertical orientation, in this example). In other examples, the flow guides 1110, 1112 may be replaced by a single flow guide that may have a configuration and a location relative to the distribution member 1100 and the header volume 122, 722 as described above.

Upon a fluid flow entering the header 100, 700 and passing through the distribution openings 1104 in the distribution member 1100, the subsequent path of the fluid flow can be restricted by the one or more flow guides 1122, in cooperation with the header wall 120, 720, to the tube region 109, 709 of the underlying tube support member 110, 710 where the inlet end array 111, 711 is exposed. This can help to more uniformly distribute the fluid flow to the tubes 106, 706 and can impede or prevent a non-beneficial flow of the fluid to the window regions 118, 718 of the tube support member 110, 710.

In some examples, the distribution member 1100 and the one or more flow guides 1122 (e.g., flow guides 1110, 1112) may be separate components. In other examples, the one or more flow guides 1122 may be affixed to or may otherwise be an integral part of the distribution member 1100. For example, the one or more flow guides 1122 may be affixed to the second side 1102b of the distribution member 1100 or the one or more flow guides 1122 may be integral portions of the distribution member 1100 that project outwardly from the second side 1102b thereof toward the tube support member 110, 710.

The distribution member 1100 differs from the distribution member 124 at least with respect to the size and the distribution of the distribution openings 1104 within the distribution region 1106 of the distribution member 1100. More specifically, unlike the distribution member 124, which has distribution openings 128 of uniform size, the distribution member 1100 has distribution openings 1104 that are non-uniform in size. In this particular example of such a distribution member 1100 configuration, the distribution openings 1104 include a plurality of what may be characterized for purposes of description, as small distribution openings 1114, medium distribution openings 1116, and large distribution openings 1118. The variety of distribution opening sizes, the opening dimensions, and frequency of occurrence of each size of distribution opening can vary in different examples.

In this example, the distribution of the distribution openings 1104 may be non-uniform across the distribution region 1106. It can be understood, particularly from the flow simulations of FIGS. 4A-4B and FIGS. 9A-9B, that the flow velocity of a fluid directed onto the distribution member 1100 will tend to be the highest at a central area 1120 of the distribution region 1106 (and the distribution member 1100) because the central area 1120 of the distribution region 1106 will underlie the header inlet 112 of the fluid processing vessel 102 or the header inlet 712 of the catalytic methanation tubular reactor 702 when the distribution member 1100 is installed thereto. Therefore, as illustrated in FIGS. 11-12, the plurality of distribution openings 1104 may be distributed such that the small distribution openings 1114 are concentrated in and near the central area 1120 of the distribution region 1106 and are not present at or near the periphery of the distribution region 1106. Likewise, the large distribution openings 1118 may be concentrated at or near the periphery of the distribution region 1106 and absent from the central area 1120 of the distribution region 1106. The medium distribution openings 1116 may, for example, be located in a transition area between the small distribution openings 1114 and the large distribution openings 1118. In other words, the sizes of the distribution openings 1104 may increase when moving from the central area 1120 toward the periphery of the distribution region 1106. The distribution member 1100 can thus equalize the flow of a fluid entering the inlet ends 106a of the tubes 106 of the fluid processing vessel 102 or the inlet ends 706a of the tubes 706 of the catalytic methanation tubular reactor 702 by reducing the fluid flow velocity through the central area 1120 of the distribution region 1106 and increasing the fluid flow velocity through the peripheral areas of the distribution region 1106.

Other distributions of the distribution openings 1104 that can allow the distribution member 1100 to produce a like effect on a fluid flow are also possible. For example, the small distribution openings 1114, the medium distribution openings 1116, and the large distribution openings 1118 may be distributed across the entirety of the distribution region 1106, but the frequency of occurrence of the small distribution openings 1114 may be greater near the central area 1120 of the distribution region 1106 while the frequency of occurrence of the large distribution openings 1118 may be greater near the periphery of the distribution region 1106.

FIG. 12 is a transparent top view of the distribution member 1100 and underlying tube support member 110, 710 arrangement shown in FIG. 11, and illustrates one example of an arrangement of the distribution openings 1104 in the distribution member 1100 relative to the underlying inlet end array 111 of the fluid processing vessel 102 or the inlet end array 711 of the catalytic methanation tubular reactor 702. As shown, the distribution openings 1104 in the distribution member 1100 may be arranged so as to surround the inlet ends 106a, 706a of the tubes 106, 706. The distribution openings 1104 overlie solid areas of the underlying tube support member 110, 710 in between the tubes 106, 706 rather than overlying the inlet ends 106a, 706a of the tubes 106, 706. This can further contribute to producing a uniform fluid flow at the inlet ends 106a, 706a of the tubes 106, 706, as fluid passing through the distribution openings 1104 is directed at a solid surface of the tube support member 110, 710 and will thus have a tendency top spread across the tube region 109, 709 of the tube support member 110, 710 instead of passing directly from a distribution opening 1104 into a tube 106, 706.

While all the distribution openings 128 in the distribution member 124 and all the distribution openings 1104 in the distribution member 1100 are shown to be of the same shape, at least some of the distribution openings may be of dissimilar shape in other distribution member examples. There is no particular limitation on the shape of the distribution openings. For example, while the distribution openings 128 in the distribution member 124 and the distribution openings 1104 in the distribution member 1100 are shown to be round in shape, the distribution openings of other distribution member examples may instead have an elliptical shape, an obround shape, or a polygonal shape.

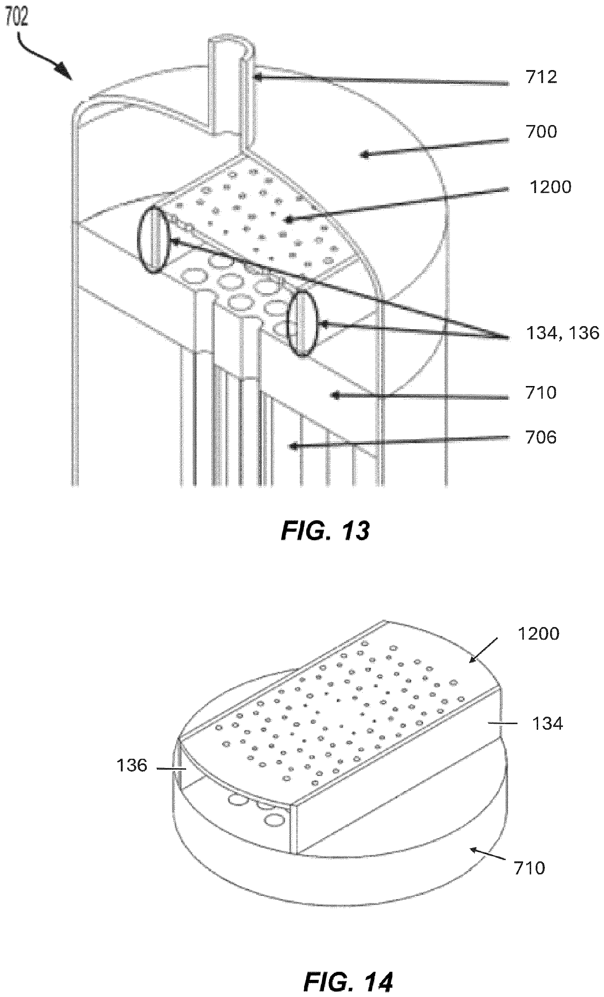

FIG 13 shows a further example an elliptical header portion 700 of a catalytic methanation tubular reactor 702 having a shell 704 and tube 706 configuration similar to the example described in FIG 7 and FIG 8. Similar reference numerals are used where applicable. FIG 14 shows only the tube support member 710, distribution member 1200, and flow guides 134, 136 of FIG 13.

In this example, only part of the peripheral shape of the distribution member 1200 matches the cross-sectional shape of the header 700 into which the distribution member 1200 will be installed. A pair of spaced apart flow guides 134, 136 extend between the distribution member 1200 and the tube support member 710 and bound the distribution openings 1204 in the distribution member 1200. Hence, opposing arcs of the periphery of the distribution member 1200 defined between the flow guides 134, 136 extend to the header wall. The spaced apart flow guides delimit the extent of the distribution member within the header volume.

A plurality of troughs (in this case two troughs) are defined in the header volume 700, one on either side of the delimited distribution member 1200. Each trough is defined by a respective flow guide 134, 136, the tube support member 710, and the inner wall of the header 700. As can be seen, each trough is in axial alignment with a respective peripheral 'no tube in window' region of the vessel.

In this example, the distribution member 1200 does not extend above the peripheral window regions, instead terminating at the point that the distribution member 1200 meets the respective flow guides 134, 136. It will be appreciated that each flow guide defines a segment within the cross-section of the header volume, the segment defined between the respective flow guide and the internal wall of the header. As can be seen, each such segment is in axial alignment with a respective peripheral 'no tube in window' region of the catalytic methanation tubular reactor.

The example of FIG. 13 and FIG 14. has been found to provide improvements in performance of the examples described with reference to FIG. 1 to FIG 12. Specifically, tests suggest that removing the solid/no aperture peripheral regions of the distribution member 1200 (that would otherwise extend over the no-window regions) can lead to reduced pressurization in the header volume and improved uniform flow in the tubes 706.

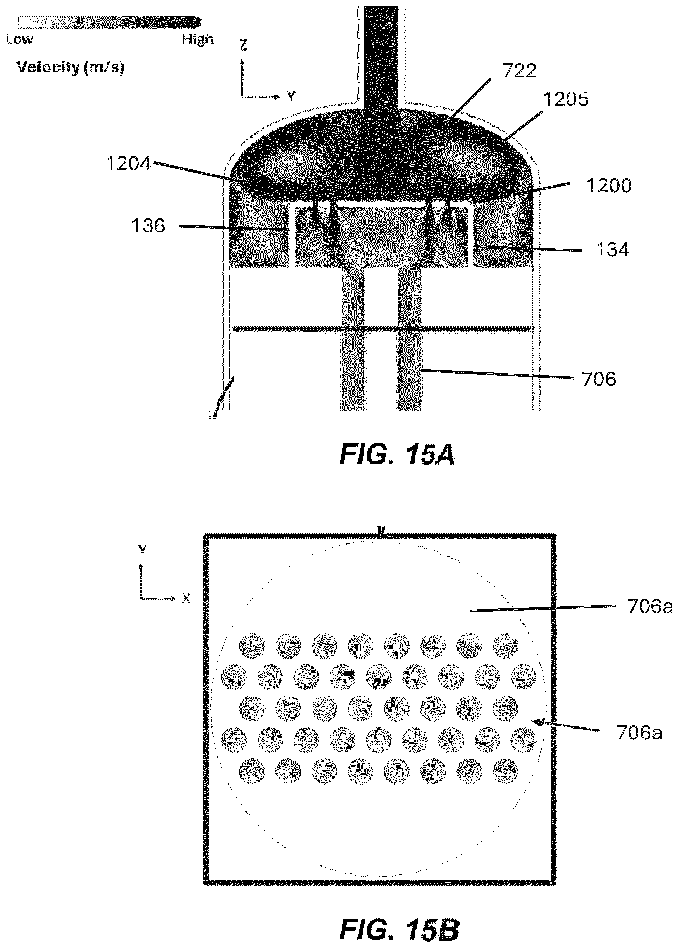

FIG. 15A shows the improved flow simulation of the example of FIG 13 and FIG 14. Compared to the flow simulation shown in FIG. 10A (for the vessel configuration of FIG. 7 and FIG. 8), there is a reduced pressurization in the header volume 722. Additionally, the high velocity portion 1204 of the fluid flow is distributed over a larger area of the tube region 709 of the tube support member 710. This is a result of the low velocity portions 1205 of the fluid flow being reduced. As may be observed in FIG. 15B, the normalized fluid flow velocity at the inlet ends 706a of the tubes 706 is uniformly distributed across substantially the entire tube region 709.

FIG. 13 is a flowchart illustrating one example of a method 1300 of equalizing the distribution of a feed gas flow across an entrance to the flow channels of a catalytic methanation tubular reactor.

As indicated in block 1302, a feed gas flow may be introduced into a header volume of the catalytic methanation tubular reactor, such as through a header inlet. In some examples, the catalytic methanation tubular reactor may have a hemispherical header. In other examples, the catalytic methanation tubular reactor may have an elliptical header (i.e. a header of elliptical cross section).

As indicated in block 1304, the feed gas flow may then be passed through a plurality of distribution openings of a distribution member positioned within the header volume. The distribution member may be located between and spaced apart from both the header inlet and an inlet end array comprising inlet ends of a plurality of flow channels that extend through a shell-side fluid flow path within a shell of the vessel. The plurality of flow channels may be a plurality of tubes.

In some examples, the distribution member may be a plate or a planar sheet and can have a peripheral shape that matches a cross-sectional shape of the header. **The** plurality of distribution openings extend through the distribution member and may be arranged along at least a portion of the distribution member to define a distribution region. **In** some examples, the distribution openings may be uniform in size and may be uniformly distributed across the distribution region. **In** other examples, the distribution openings may be uniform in size but non-uniformly distributed across the distribution region. **In** still other examples, the distribution openings may be non-uniform in size and the size of the distribution openings may increase as the distance from the header inlet increases. **In** some examples, the distribution openings may all have the same shape, while in other examples, at least some of the distribution openings may be of dissimilar shape.

As indicated in block 1306, the feed gas flow can be directed from the distribution member to the inlet end array through a narrowed portion of the header volume. The narrowed portion of the header volume may be defined at least in part by one or more flow guides that extend between the distribution member and the inlet end array. For example, the one or more flow guides may extend between the distribution member and a flow channel (e.g., tube) support member in which the inlet end array comprising the inlet ends of the flow channels is retained. The one or more flow guides can at least partially bound the distribution openings in the distribution member and the inlet ends of the plurality of flow channels (e.g., tubes).

In some examples, the one or more flow guides may comprise a pair of spaced apart flow guides. In some examples, the one or more flow guides may comprise a single flow guide. In some examples, the distribution member and the one or more flow guides may be separate components. In other examples, the one or more flow guides may be affixed to or may otherwise be an integral part of the distribution member. A height dimension of the one or more flow guides may define a distance of separation of the distribution member from the flow channel support member and the inlet end array exposed therein. At least when the one or more flow guides is a pair of spaced apart flow guides, the flow guides may also extend along the second side of the distribution member such that opposite ends of each flow guide abut the inner wall of the header.

Passing the feed gas flow through the distribution openings in the distribution member and thereafter through the narrowed portion of the header volume defined by the one or more flow guides can more uniformly distribute the feed gas flow across the inlet ends of the flow channels. More specifically, use of the distribution member in combination with the one or more flow guides may operate to provide a more uniform feed gas flow rate through the flow channels (e.g., tubes). For example, the distribution openings may operate to equalize the feed gas flow velocity through the distribution member by slowing the velocity of the feed gas flow through a central portion of the distribution member that underlies or is closer to the header inlet. This can allow the velocity of the feed gas flow through the central portion of the distribution member to better match the velocity of the feed gas flow through a portion of the distribution member that is farther away from the header inlet. Use of the flow guides can, for example, restrict the flow of feed gas passing through the distribution member to the region of the underlying flow channel support member that contains the inlet end array. This can also improve the uniformity of the feed gas flow rate distribution at the inlet ends of and through the flow channels.

The foregoing description of certain examples, including illustrated examples, has been presented only for the purpose of illustration and description and is not intended to be exhaustive or to limit the disclosure to the precise forms disclosed. Numerous modifications, adaptations, and uses thereof will be apparent to those skilled in the art without departing from the scope of the disclosure.

## Claims

1. A catalytic methanation reactor comprising:
a header comprising a header inlet and a header wall, the header wall defining a header volume in fluid communication with the header inlet;
a plurality of fluid flow channels extending downstream from a first support member residing in the header volume, the plurality of fluid flow channels in fluid communication with the header volume at inlet openings of the fluid flow channels, the inlet openings arranged in an inlet opening array;
a distribution member located in the header volume and comprising a plurality of distribution openings, the distribution member located between and spaced apart from both the header inlet and the inlet opening array;
a flow guide extending between the inlet opening array and the distribution member, the flow guide narrowing a portion of the header volume between the distribution member and the inlet opening array; and
a catalyst located in at least some of the plurality of fluid flow channels to facilitate a reaction of a feed gas that is flowable through the at least some of the plurality of fluid flow channels.

2. The catalytic methanation reactor of claim 1, wherein catalytic methanation reactor is intended to be orientated to be substantially vertical with the distribution member above the first support member, and wherein fluid is intended to flow downwards through the distribution member and into the inlet openings of the fluid flow channels so as to flow down along a longitudinal axis of the fluid flow channels.

3. The catalytic methanation reactor of claim 1, wherein the flow guide is axially parallel with a longitudinal axis of the fluid flow channels and extends between the distribution member and the first support member.

4. The catalytic methanation reactor of claim 1, wherein the flow guide defines a wall which extends across the header volume and has opposing longitudinal ends, wherein each longitudinal end is in contact with the header wall; optionally, wherein the reactor comprises a pair of flow guides, wherein each flow guide defines a wall which extends across the header volume and has opposing longitudinal ends, wherein each longitudinal end is in contact with the header wall.

5. The catalytic methanation reactor of claim 1, wherein the fluid flow channels are tubes and the reactor has a no-tube-in-window configuration where the inlet opening array resides in a tube region of the first support member that is aligned with the header inlet and is bounded on two opposed sides by window regions having no tubes.

6. The catalytic methanation reactor of claim 1, wherein a trough is defined in the header volume between the flow guide, first support member and the header wall; optionally, wherein the trough is in axial alignment with a no-tube-in-window region of the first support member, optionally wherein the inlet opening array comprises a first planar array of openings, wherein the distribution openings are arranged in a second planar array of openings, and wherein the flow guide comprises at least one wall extending between the first and second planar arrays, optionally wherein the support member is a planar member, the first planar array of openings is located in the support member, and the distribution member is a plate or a planar sheet, optionally wherein:
the support member comprises a first central region comprising the first planar array of openings and at least one first peripheral region in which no inlet openings are located, wherein the at least one wall extends from a boundary between the first central region and the first peripheral region; and
the distribution member comprises a second central region comprising the distribution openings and at least one second peripheral region in which no distribution openings are located, wherein the at least one wall extends from a boundary between the second central region and the second peripheral region, optionally wherein the plurality of distribution openings in the distribution member are all uniform in size and are distributed uniformly across the second central region of the distribution member; or at least some of the plurality of distribution openings in the distribution member are non-uniform in size; distribution openings of a smaller size are concentrated near a central area of the second central region of the distribution member; and distribution openings of a larger size are concentrated near a periphery of the second central region of the distribution member, optionally wherein the fluid flow channels are tubes and the support member is a tube sheet.

7. The catalytic methanation reactor of claim 1, wherein the header is an elliptical header.

8. A catalytic methanation tubular reactor comprising:
a shell defining a shell-side fluid flow path;
a plurality of tubes at least partially extending through the shell-side fluid flow path, each tube comprising an inlet end, the inlet ends arranged in an inlet end array located in a tube support member;
an elliptical header comprising a header inlet, and a header wall at least partially defining a header volume, the header volume in fluid communication with the inlet end array;
a distribution member comprising a plurality of distribution openings, and positioned in the header volume between the header inlet and the tube support member so as to be spaced apart from the inlet end array;
one or more flow guides extending between the distribution member and the tube support member and at least partially bounding the distribution openings in the distribution member and the inlet ends of the plurality of tubes; and
a catalyst located in at least some of the plurality of tubes to facilitate a reaction of a feed gas that is flowable through the at least some of the plurality of tubes;
optionally, wherein the fluid processing vessel is intended to be orientated to be substantially vertical with the distribution member above the support member, and wherein fluid is intended to flow downwards through the distribution member and into the inlet ends of the tubes of the flow channels so as to flow down along a longitudinal axis of the tubes.

9. The catalytic methanation reactor of claim 8, wherein the tube support member is a tube sheet having a no-tube-in-window configuration where the inlet end array resides in a tube region of the tube sheet that is aligned with the header inlet and is bounded on two opposed sides by window regions having no tubes.

10. The catalytic methanation reactor of claim 8, wherein the one or more flow guides are axially parallel with a longitudinal axis of the fluid flow channels and extend between the distribution member and the first support member.

11. The catalytic methanation reactor of claim 8, wherein each flow guide defines a wall which extends across the header volume and has opposing longitudinal ends, and wherein each longitudinal end is in contact with the header wall.

12. The catalytic methanation reactor of claim 8, wherein the one or more flow guides comprises a pair of spaced apart flow guides, wherein the spaced apart flow guides delimit the extent of the distribution member within the header volume, and define a pair of spaced apart troughs, each trough defined in the header volume between a respective flow guide, the first support member and the header wall.

13. A method comprising:
introducing a feed gas flow into a header volume of a header of a catalytic methanation reactor;
passing the feed gas flow through a plurality of distribution openings of a distribution member positioned within the header volume and located between and spaced apart from both a header inlet and an inlet end array comprising inlet ends of a plurality of flow channels that extend through a shell-side fluid flow path within a shell of the catalytic methanation reactor;
directing the feed gas flow from the distribution member to the inlet end array through a narrowed portion of the header volume defined at least in part by one or more flow guides that extend between the distribution member and the inlet end array and at least partially bound the distribution openings in the distribution member and the inlet ends of the plurality of flow channels; and
generating methane by contacting the feed gas flow with a catalyst located in at least some of the plurality of flow channels.

14. The method of claim 13, wherein the catalytic methanation reactor is substantially vertical with the distribution member above the inlet end array, and wherein fluid is intended to flow downwards through the distribution member and into the inlet ends of the flow channels so as to flow down along a longitudinal axis of the flow channels; wherein the one or more flow guides are axially parallel with a longitudinal axis of the fluid flow channels and extend between the distribution member and the inlet end array.

15. The method of claim 13, wherein:
the feed gas is a mixture comprising at least carbon monoxide and hydrogen, carbon dioxide and hydrogen, or carbon monoxide, carbon dioxide, and hydrogen; and
the catalyst is a metal selected from the group consisting of nickel, ruthenium, rhodium, and palladium.
